# FASCICULE DE BREVET EUROPEEN

(11) **EP 0 571 264 B1**
(45) Date de publication et mention de la délivrance du brevet: **25.11.1998**
(21) Numéro de dépôt: 93401262.6
(22) Date de dépôt: 18.05.1993
(51) Int. Cl.: A61K 31/505, A61K 9/70

(54) **Système matriciel autoadhésif pour la libération prolongée du piribédil par voie transcutanée**
Selbstklebendes Matrixsystem für die transdermal-gesteuerte Abgabe von Piribedil
Autoadhesive matrix system for the transdermal controlled release of piribedil

(30) Priorité: 19.05.1992 FR 9206037
(43) Date de publication de la demande: 24.11.1993
(73) Titulaire: ADIR ET COMPAGNIE, 92415 Courbevoie Cédex (FR)
(72) Inventeur: Cuine, Alain, F-45800 Saint Jean de Braye (FR); Rault, Isabelle, F-45170 Saint Lye la Foret (FR); Arnaud, Françoise, F-92250 La Garenne Colombes (FR); Crambes, Olivier, F-91360 Marolles en Hurepoix (FR); Pichon, Gérald, F-45100 Orleans (FR)

(56) Documents cités:
- EP-A- 0 468 875
- FR-A- 2 355 502

## Description

La présente invention a pour objet un système matriciel autoadhésif pour la libération prolongée du piribédil qui assure la diffusion continue et progressive du principe actif par voie transcutanée.

Le piribédil, composé de formule (I): 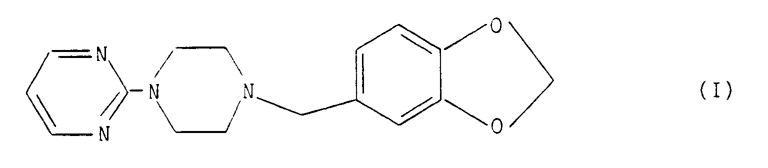 est un agoniste dopaminergique qui stimule les récepteurs à la dopamine et les voies dopaminergiques cérébrales et périphériques.

Le piribédil était jusqu'alors administré par voie orale ou injectable.

L'administration de ce principe actif par voie transcutanée présente l'avantage au plan pharmacocinétique d'une obtention rapide de taux plasmatiques élevés et constants, au plan métabolique d'éviter la métabolisation importante du principe actif par effet de premier passage hépatique et enfin au plan clinique de diminuer les doses administrées en améliorant l'efficacité, l'acceptabilité clinique et l'observance par une seule application non traumatisante.

Les possibilités d'administration d'un médicament à travers la peau dépendent de différents facteurs. En particulier, le composé ne doit pas altérer la peau d'une manière quelconque à la suite d'un contact prolongé et provoquer irritations, allergies ou sensibilisations, et doit pouvoir traverser une surface de peau assez petite à un débit de diffusion suffisant pour l'obtention de taux plasmatiques adaptés aux besoins thérapeutiques.

Or, le piribédil satisfait à toutes ces exigences.
Plus spécifiquement, la présente invention a pour objet un système matriciel autoadhésif qui permet la libération prolongée du piribédil et son passage par voie transcutanée à travers une surface de peau déterminée, pendant une période prolongée et à un débit suffisant pour provoquer la stimulation des récepteurs dopaminergiques.

Ainsi le système transdermique de piribédil permet d'améliorer les symptômes du déficit intellectuel pathologique du sujet âgé. Il est également utile dans le traitement d'appoint de la claudication intermittente des artériopathies chroniques oblitérantes des membres inférieurs, dans le traitement de la maladie de Parkinson en monothérapie ou en association à la dopathérapie, dans les sensations d'étourdissement du sujet âgé, dans les accidents ischémiques rétiniens et cochléovestibulaires, dans les accidents vasculaires cérébraux ainsi que dans les manifestations aiguës de l'artérite des membres inférieurs.

Depuis quelques années, de nombreux brevets décrivent des systèmes pour l'administration de médicaments par voie transdermique à la circulation générale.

En particulier, il existe des sytèmes autoadhésifs dans lesquels le principe actif est directement dissous dans l'adhésif.

D'autre part, il existe des systèmes matriciels dans lesquels le principe actif est à la fois dissous et dispersé à l'état particulaire ; la préparation est alors rendue adhésive par l'enduction d'un adhésif en surface ou par un anneau situé autour de la matrice.

Un système matriciel autoadhésif pour la libération prolongée du piribédil par voie transcutanée a déjà été décrit dans la demande FR 2664815. Le système matriciel autoadhésif décrit dans la présente invention, outre le fait qu'il soit nouveau, présente l'avantage de permettre une meilleure libération du piribédil et donc d'atteindre des taux plasmatiques en principe actif nettement plus élevés que ceux obtenus avec le système décrit dans la demande FR 2664815. D'autre part, les copolymères utilisés dans ce nouveau système ont un prix de revient environ dix fois moins élevé ce qui représente un avantage industriel tout à fait notable.

Ce système matriciel dont la matrice est composée pour sa partie non adhésive d'éthylène/acétate de vinyle est de conception nouvelle.

La libération du piribédil se fait de façon continue et constante pendant toute la durée d'application du système sur la peau.

Le dispositif transdermique est constitué d'un film support occlusif ou non, enduit d'une matrice polymère autoadhésive, recouverte éventuellement d'une seconde couche principalement constituée d'adhésif et de copolymère d'éthylène/acétate de vinyle. L'ensemble est protégé par un film protecteur pelable.

La matrice proprement dite est constituée d'un réseau polymère d'éthylène/acétate de vinyle mélangé à un adhésif. Cette matrice est donc autoadhésive ce qui permet au système de se maintenir en place pendant une période allant de 1 à 7 jours.

Le squelette de la matrice polymère est constitué d'un copolymère d'éthylène/acétate de vinyle de qualité médicale de formule générale : 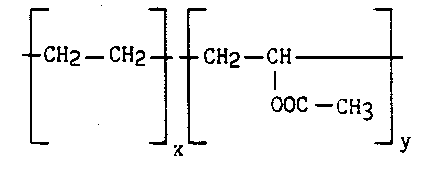
- dans laquelle x :: 60 à 95 % en poids
- et y :: 40 à 5 % en poids.

La matrice peut être composée d'un ou plusieurs types de copolymères d'éthylène/acétate de vinyle. La variation de la composition ayant pour buts principaux de modifier la souplesse et les propriétés "adhérence instantanée" de la matrice polymère.

Le pouvoir autoadhésif de la matrice est conféré par l'incorporation d'un adhésif de type acrylique en solution dans un solvant organique approprié comme le dichlorométhane, l'heptane, l'acétate d'éthyle, le 1,3-dioxolane, les solvants de remplacement des solvants chlorés comme le 1,1-dichloro-1-fluoroéthane, à une concentration déterminée et à chaud si nécessaire. Les propriétés de l'ensemble sont révélées par évaporation du solvant.

L'adhésif de type acrylique peut être remplacé par un adhésif de type polybutilène, métacrylate, silicone ou par une émulsion acrylique dans un solvant organique approprié.

Des promoteurs d'absorption peuvent être ajoutés à la matrice. Ces promoteurs d'absorption peuvent être des acides gras et leurs dérivés, des glycérides polyglycosilés saturés ou insaturés, des oléates, du diéthylène glycol monoéthylether et plus spécifiquement du diméthylisosorbide, du propylène glycol, des terpènes. Un ou plusieurs de ces composés peuvent être incorporés dans la matrice. Le taux d'incorporation de ces promoteurs d'absorption cutanée dans la forme transdermique peut varier selon l'efficacité du (des) promoteur(s) employé(s).

Les quantités de piribédil dans la matrice varient en fonction des doses nécessaires pour un type de patient particulier et sont comprises entre 10 et 500 mg. Le principe actif peut être utilisé sous forme micronisée ou non. La surface des dispositifs peut varier de 5 à 80 cm².

Les exemples suivants illustrent l'invention mais ne la limitent en aucune façon.

### Exemple 1 :

35 g de copolymère d'éthylène/acétate de vinyle (40 % d'acétate) sont dissous dans environ 200 ml de dichlorométhane.
Séparément on pèse 5 g de piribédil (sous forme micronisée) auxquels sont ajoutés 20 g d'acide oléique. Le mélange est effectué à l'aide d'ultrasons. Cette solution est mélangée au copolymère dissous.
40 g d'adhésif acrylique sont alors ajoutés au mélange précédent. Le mélange est ensuite enduit sur un film support occlusif en polyéthylène/aluminium au moyen d'un étaleur permettant de régler l'épaisseur.
La structuration de l'ensemble s'effectue par évaporation de solvant à 80°C.
Un film protecteur pelable en polyester est appliqué sur le système adhésif réticulé.
La matrice autoadhésive est alors découpée de façon à obtenir des dispositifs de surface de 20 cm² comprenant une quantité de principe actif égale à 5 % de la masse totale du dispositif transdermique.

### Exemple 2 :

52,5 g de copolymère d'éthylène/acétate de vinyle (40 % d'acétate) sont dissous dans environ 270 ml de dichlorométhane.
Séparément on pèse 7,5 g de piribédil (sous forme micronisée) auxquels sont ajoutés 20 g d'acide oléique. D'après le protocole décrit dans l'exemple 1 sont ajoutés ensuite 20 g d'adhésif acrylique.
Les dispositifs sont obtenus de la même façon que dans l'exemple 1. La quantité de principe actif est égale à 7,5 % de la masse totale du dispositif transdermique.

### Exemple 2 bis :

52,5 g de copolymère d'éthylène/acétate de vinyle (40 % d'acétate) sont dissous dans environ 500 ml d'acétate d'éthyle.
Séparément, on pèse 7,5 g de piribédil (sous forme micronisée) auxquels sont ajoutés 20 g d'acide oléique. D'après le protocole décrit dans l'exemple 1 sont ajoutés ensuite 20 g d'adhésif acrylique.

Les dispositifs sont obtenus de la même façon que dans l'exemple 1 mais, dans ce cas, le solvant est évaporé à 50°C. La quantité de principe actif est égale à 7,5 % de la masse totale du dispositif transdermique.

### Exemple 3 :

57,5 g de copolymère d'éthylène/acétate de vinyle (40 % d'acétate) sont dissous dans environ 500 ml d'acétate d'éthyle.
Séparément, on pèse 7,5 g de piribédil (sous forme micronisée) auxquels sont ajoutés 15 g d'acide oléique et 5 g de propyléne glycol. D'après le protocole décrit dans l'exemple 1 sont ajoutés ensuite 15 g d'adhésif acrylique.
Les dispositifs sont obtenus de la même façon que dans l'exemple 1 mais, dans ce cas, le solvant est évaporé à 50°C. La quantité de principe actif est égale à 7,5 % de la masse totale d'un dispositif transdermique.

### Exemple 4 :

35 g de copolymère d'éthylène/acétate de vinyle (40 % d'acétate) sont dissous dans environ 300 ml d'acétate d'éthyle.

Séparément, on pèse 5 g de piribédil (sous forme micronisée) auxquels sont ajoutés 20 g d'acide oléique. Sont ajoutés ensuite 15 g d'adhésif acrylique.
Les dispositifs sont obtenus de la même façon que dans l'exemple 1 mais, dans ce cas, le solvant est évaporé à 50°C. La quantité de principe actif est égale à 5 % de la masse totale du dispositif transdermique.

### Exemple 5 :

52,5 g de copolymère d'éthylène/acétate de vinyle (40 % d'acétate) sont dissous dans environ 400 ml de 1,1-dichloro-1-fluoroéthane.
Selon le protocole décrit dans l'exemple 1 sont ensuite ajoutés 7,5 g de piribédil puis 20 g d'acide oléique et enfin 6 g d'adhésif acrylique.
Les dispositifs sont obtenus de la même façon que dans l'exemple 1 mais dans ce cas, le solvant est évaporé à 30°C. La quantité de principe actif dans le dispositif transdermique est égale à 7,5 % de sa masse totale .

### Exemple 6 :

35 g de copolymère d'éthylène/acétate de vinyle (40 % d'acétate) sont dissous dans environ 180 ml de dichlorométhane. Selon le protocole décrit dans l'exemple 1 sont ensuite ajoutés 5 g de piribédil puis 20 g d'acide oléique et enfin 40 g d'élastomère silicone de qualité médicale (X7 4301/Dow Corning à 60 % dans le dichlorométhane).
La structuration du mélange enduit sur le film polyéthylène/aluminium/polyester est effectuée à la chaleur sous un rayonnement infra-rouge.
La quantité de principe actif dans le dispositif transdermique est égale à 5 % de la masse totale du dispositif.

### Exemple 7 :

35 g de copolymère d'éthylène/acétate de vinyle (40 % d'acétate) sont dissous dans environ 180 ml de dichlorométhane. Selon le protocole décrit dans l'exemple 1 sont ensuite ajoutés 5 g de piribédil puis 20 g d'acide oléique et enfin 40 g d'adhésif de type polyisobutène (Oppanol/BASF dans le dichlorométhane).

La réticulation est réalisée par passage à l'étuve à 80°C pendant environ un quart d'heure.
La quantité de principe actif dans le dispositif transdermique est égale à 5 % de sa masse totale.

### Exemple 8 :

35 g de copolymère d'éthylène/acétate de vinyle (40 % d'acétate) sont dissous dans environ 200 ml de dichlorométhane. Selon le protocole décrit dans l'exemple 1 sont ensuite ajoutés 5 g de piribédil puis 20 g d'acide oléique et enfin 40 g d'adhésif de type methacrylate (Eudragit 2780 D-L/RHOM-PHARMA dans du dichlorométhane).
La réticulation est réalisée par passage à l'étuve à 80°C pendant environ un quart d'heure.
La quantité de principe actif dans le dispositif transdermique est égale à 5 % de sa masse totale.

### Exemple 9 :

Etape 1 : 62,5 g de copolymère d'éthylène/acétate de vinyle (40 % d'acétate) sont dissous dans environ 200 ml d'acétate d'éthyle à une température comprise entre 60 et 70°C. Séparément, on pèse 7,5 g de piribédil (sous forme non micronisée) auxquels sont ajoutés 20,0 g d'acide oléique. D'après le protocole décrit dans l'exemple 1 sont ensuite ajoutés 10,0 g d'adhésif acrylique. Le mélange est ensuite enduit sur un support occlusif en polyéthylène/aluminium au moyen d'un étaleur permettant de régler l'épaisseur de la couche de mélange enduite. La structuration de l'ensemble s'effectue par évaporation progressive du solvant en faisant varier la température de 20°C à 50°C.
Etape 2 : 4,5 g de copolymère d'éthylène/acétate de vinyle (40 % d'acétate) sont dissous dans environ 40 ml d'acétate d'éthyle à une température comprise entre 60 et 70°C. Séparément, on pèse 0,5 g de piribédil non micronisé auxquels sont ajoutés 0,5 g d'acide oléique. Le mélange est effectué à l'aide d'ultra-sons. Cette solution est mélangée à la solution de copolymère dissous. Sont ensuite ajoutés 4,5 g d'adhésif acrylique. Le mélange est enduit sur la couche réalisée durant la première étape à l'aide d'un étaleur permettant l'application d'un film de faible épaisseur. Le solvant est ensuite progressivement évaporé en faisant varier la température de 30°C à 90°C. L'ensemble est refroidi à température ambiante durant quelques heures. Un film protecteur siliconé pelable est appliqué sur le système. La masse autoadhésive est alors découpée de façon à obtenir des dispositifs de surface variant de 5 cm2 à 80 cm2 comprenant une quantité de principe actif égale à 7,5 % de la masse totale du dispositif transdermique.

### Exemple 10 :

La fabrication décrite dans l'exemple 9 peut être réalisée par une méthode différente dite "méthode de transfert". L'étape 1 est identique à celle décrite dans l'exemple 9. Le mélange est dans ce cas enduit sur un film protecteur siliconé intermédiaire puis recouvert d'un film polyéthylène/aluminium. Le mélange issu de l'étape 2 (identique à celle décrite dans l'exemple 9) est enduit sur un film protecteur siliconé et recouvert par un film protecteur siliconé intermédiaire. Dans une troisième étape on réalise le transfert des deux couches après avoir enlevé les deux films protecteurs intermédiaires.

### Etude pharmacologique des produits de l'invention

### Exemple 11 : Etude de la libération du piribédil par voie transdermique à travers de la peau de rat

L'étude de la libération du piribédil par voie transcutanée a été réalisée en cellule de Franz sur peau de rat Hairless. La phase réceptrice est un tampon citrate (pH = 4,5).
Les résultats comparant le système décrit dans l'exemple 2 bis et celui décrit dans l'exemple 4 du brevet FR 2664815 ont été rassemblés dans le schéma 1 (annexe). L'allure des deux courbes de ce schéma montre que le système de la présente invention permet d'augmenter considérablement la quantité de piribédil libérée dès la première demi-heure d'application du système.

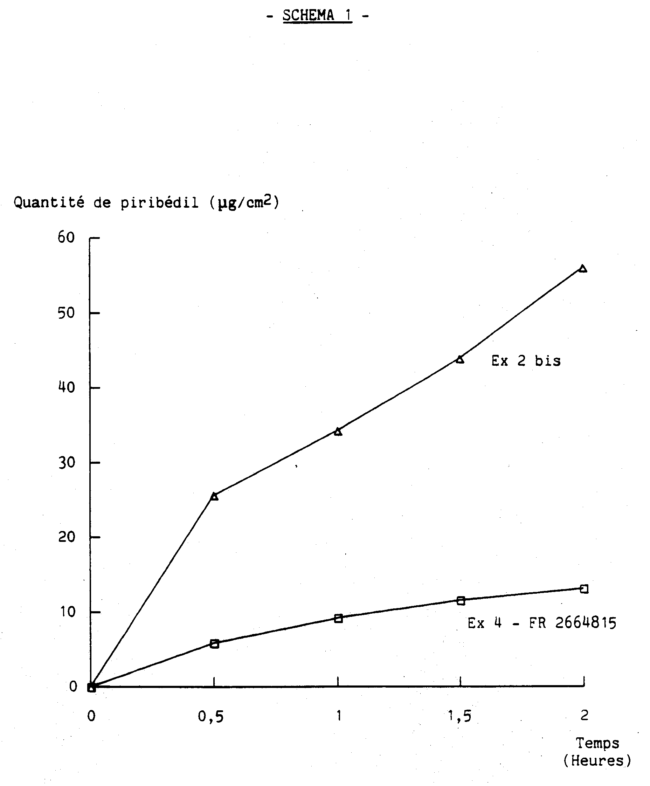

## Revendications

1. Système matriciel autoadhésif pour la libération prolongée du piribédil par voie transcutanée caractérisé en ce qu'il comprend un film support occlusif ou non enduit d'une matrice autoadhésive dont le squelette polymère est constitué d'un polymère éthylène/acétate de vinyle de qualité médicale recouverte éventuellement d'une seconde couche principalement constituée d'adhésif et de copolymère éthylène/acétate de vinyle et protégée par un film protecteur pelable.

2. Système matriciel autoadhésif de piribédil selon la revendication 1 caractérisé en ce que la matrice autoadhésive contient un ou plusieurs promoteurs d'absorption.

3. Système matriciel autoadhésif selon la revendication 2 caractérisé en ce que les promoteurs d'absorption contenu dans la matrice autoadhésive sont des acides gras et leurs dérivés, a des glycérides polyglycosilés saturés ou insaturés.

4. Système matriciel autoadhésif de piribédil selon la revendication 2 caractérisé en ce que l'un au moins des promoteurs d'absorption est de l'acide oléique.

5. Système matriciel autoadhésif de piribédil selon l'une quelconques des revendications 1 ou 2, caractérisé en ce que l'autoadhésivité de ladite matrice est obtenue par la mise en solution d'un adhésif dans un solvant organique.

6. Système matriciel autoadhésif de piribédil selon la revendication 5 caractérisé en ce que l'adhésif est de type acrylique.

7. Système matriciel autoadhésif selon l'une quelconque des revendications 1 à 6 pour une utilisation dans le traitement des symptômes du déficit intellectuel pathologique du sujet âgé, dans le traitement d'appoint de la claudication intermittente des artériopathies chroniques oblitérantes des membres inférieurs, dans le traitement de la maladie de Parkinson en monothérapie ou en association avec d'autres thérapeutiques à effet synergique, dans les sensations d'étourdissement du sujet âgé, dans les accidents ischémiques rétiniens et cochléovestibulaires, dans les accidents vasculaires cérébraux ainsi que dans les manifestations aiguës de l'artérite des membres inférieurs.

## Claims

1. Self-adhesive matrix system for the sustained release of piribedil by the transcutaneous route, characterised in that it comprises an occlusive or non-occlusive carrier film coated with a self-adhesive matrix, the polymer skeleton of which is composed of a medical-grade ethylene/vinyl acetate polymer, optionally covered by a second layer composed principally of adhesive and ethylene/vinyl acetate copolymer and protected by a peelable protective film.

2. Self-adhesive matrix system of piribedil according to claim 1, characterised in that the self-adhesive matrix comprises one or more absorption promoters.

3. Self-adhesive matrix system according to claim 2, characterised in that the absorption promoters present in the self-adhesive matrix are fatty acids or derivatives thereof, or saturated or unsaturated polyglycosylated glycerides.

4. Self-adhesive matrix system of piribedil according to claim 2, characterised in that at least one of the absorption promoters is oleic acid.

5. Self-adhesive matrix system of piribedil according to either claim 1 or claim 2, characterised in that the self-adhesiveness of the said matrix is obtained by dissolving an adhesive in an organic solvent.

6. Self-adhesive matrix system of piribedil according to claim 5, characterised in that the adhesive is of the acrylic type.

7. Self-adhesive matrix system according to any one of claims 1 to 6 for use in the treatment of symptoms of pathological intellectual deficit in the elderly patient, in the ancillary treatment of intermittent claudication in chronic occlusive arteriopathies in the lower limbs, in the treatment of Parkinson's disease as the sole therapeutic agent or in association with other therapeutic agents having a synergistic effect, in sensations of giddiness in the elderly patient, in retinal and cochleovestibular ischaemic incidents, in cerebrovascular accidents and also in acute manifestations of arteritis in the lower limbs.

## Patentansprüche

1. Selbstklebendes Matrixsystem für die verzögerte Freisetzung von Piribedil auf transkutanem Wege, dadurch gekennzeichnet, daß es einen gegebenenfalls okklusiven Trägerfilm umfaßt, der mit einer selbstklebenden Matrix beschichtet ist, deren Polymergerüst durch ein Ethylen/Vinylacetat-Polymer von medizinischer Qualität gebildet ist, welche gegebenenfalls mit einer zweiten im wesentlichen aus Klebstoff und Ethylen/Vinylacetat-Copolymer gebildeten Schicht bedeckt ist und mit einem abziehbaren Schutzfilm geschützt ist.

2. Selbstklebendes Matrixsystem für Piribedil nach Anspruch 1, dadurch gekennzeichnet, daß die selbstklebende Matrix einen oder mehrere Absorptions-Promotoren enthält.

3. Selbstklebendes Matrixsystem nach Anspruch 2, dadurch gekennzeichnet, daß die in der selbstklebenden Matrix enthaltenen Absorptions-Promotoren Fettsäuren und deren Derivate oder gesättigte oder ungesättigte polyglykosylierte Glyceride sind.

4. Selbstklebendes Matrixsystem für Piribedil nach Anspruch 2, dadurch gekennzeichnet, daß mindestens einer der Absorptions-Promotoren Ölsäure ist.

5. Selbstklebendes Matrixsystem für Piribedil nach einem der Ansprüche 1 oder 2, dadurch gekennzeichnet, daß das Selbstklebevermögen der Matrix durch Lösen eines Klebstoffs in einem organischen Lösungsmittel erzeugt wird.

6. Selbstklebendes Matrixsystem für Piribedil nach Anspruch 5, dadurch gekennzeichnet, daß der Klebstoff ein Klebstoff des Acryl-Typs ist.

7. Selbstklebendes Matrixsystem nach einem der Ansprüche 1 bis 6 zur Verwendung bei der Behandlung von Symptomen von pathologischen intellektuellen Mangelerscheinungen, bei der ergänzenden Behandlung von intermittierenden Gehstörungen bei chronischen verschließenden Arteriopathien der unteren Glieder, bei der Behandlung der Parkinsonschen Erkrankung durch Monotherapie oder in Kombination mit anderen Behandlungsformen mit synergischer Wirkung, bei Schwindelgefühlen bei älteren Patienten, bei Retina- und Ohrschneckenvorhof-bezüglichen ischämischen Vorfällen, bei Zerebralgefäßvorfällen sowie akuten Manifestationen der Arteriitis der unteren Glieder.
